# EUROPÄISCHE PATENTSCHRIFT

(11) **EP 3 764 797 B1**
(45) Veröffentlichungstag und Bekanntmachung des Hinweises auf die Patenterteilung: **30.03.2022**
(21) Anmeldenummer: 19718236.3
(22) Anmeldetag: 12.03.2019
(51) Int. Cl.: A01N 63/38, A01P 21/00, C12Q 1/6895, C12Q 1/686

(54) **PILZSTAMM DER GATTUNG TRICHODERMA UND VERFAHREN ZUR FÖRDERUNG VON PFLANZENWACHSTUM**
FUNGAL STRAIN OF THE GENUS TRICHODERMA AND METHOD FOR THE PROMOTION OF PLANT GROWTH
SOUCHE FONGIQUE DU GENRE TRICHODERMA ET PROCÉDÉ DE PROMOTION DE LA CROISSANCE DES PLANTS

(30) Priorität: 15.03.2018 DE 102018002234
(43) Veröffentlichungstag der Anmeldung: 20.01.2021
(73) Patentinhaber: Hochschule Anhalt, 06366 Köthen (DE)
(72) Erfinder: SCHELLENBERG, Ingo, 06847 Dessau-Rosslau (DE); GEISTLINGER, Jörg, 06458 Heteborn (DE)
(74) Vertreter: Fischer, Volker
(86) Internationale Anmeldenummer: PCT/DE2019/000074
(87) Internationale Veröffentlichungsnummer: WO 2019/174664

(56) Entgegenhaltungen:
- EP-A1- 1 279 335
- WO-A1-2010/091337
- WO-A1-2011/032281
- WO-A2-2004/089831
- CN-A- 102 505 010
- US-B1- 6 475 772
- US-B1- 6 808 917
- U. YADAV ET AL: "Biodegradation of sulfosulphuron in agricultural soil by Trichoderma sp.", LETTERS IN APPLIED MICROBIOLOGY, Bd. 59, Nr. 5, 13. August 2014 (2014-08-13), Seiten 479-486, XP55593334, GB ISSN: 0266-8254, DOI: 10.1111/lam.12306
- DOMINIK SKONECZNY ET AL: "Genetic diversity of Trichoderma atroviride strains collected in Poland and identification of loci useful in detection of within-species diversity", FOLIA MICROBIOLOGICA., Bd. 60, Nr. 4, 20. März 2015 (2015-03-20), Seiten 297-307, XP55593416, NL ISSN: 0015-5632, DOI: 10.1007/s12223-015-0385-z

### SEQUENCE LISTING

<110> Hochschule Anhalt
<120> Pilzstamm der Gattung Trichoderma und Verfahren zur Förderung von Pflanzenwachstum
<130> HSA22-17-IP
<150> DE 10 2018 002 234.0
   <151> 2018-03-15
<160> 45
<170> PatentIn version 3.5
<210> 1
   <211> 20
   <212> DNA
   <213> Trichoderma sp. 'HSA12'
<400> 1
   cggatgtgag acgcaatatg 20
<210> 2
   <211> 20
   <212> DNA
   <213> Trichoderma sp. 'HSA12'
<400> 2
   caacagcgaa gtgttgatgg 20
<210> 3
   <211> 20
   <212> DNA
   <213> Trichoderma sp. 'HSA12'
<400> 3
   tcaacttcgc cctcattttc 20
<210> 4
   <211> 20
   <212> DNA
   <213> Trichoderma sp. 'HSA12'
<400> 4
   cgatctcgaa gctgacacag 20
<210> 5
   <211> 20
   <212> DNA
   <213> Trichoderma sp. 'HSA12'
<400> 5
   gtctggctac attggccttc 20
<210> 6
   <211> 20
   <212> DNA
   <213> Trichoderma sp. 'HSA12'
<400> 6
   agacggaggg ggagattatg 20
<210> 7
   <211> 20
   <212> DNA
   <213> Trichoderma sp. 'HSA12'
<400> 7
   tcctcctcaa tcacctttgc 20
<210> 8
   <211> 20
   <212> DNA
   <213> Trichoderma sp. 'HSA12'
<400> 8
   tttcccgaag aaatcacagg 20
<210> 9
   <211> 20
   <212> DNA
   <213> Trichoderma sp. 'HSA12'
<400> 9
   gccacagaga gaagccagtc 20
<210> 10
   <211> 20
   <212> DNA
   <213> Trichoderma sp. 'HSA12'
<400> 10
   gcgtcatgtc cccatctatc 20
<210> 11
   <211> 20
   <212> DNA
   <213> Trichoderma sp. 'HSA12'
<400> 11
   tttcttcgtg tttccccatc 20
<210> 12
   <211> 20
   <212> DNA
   <213> Trichoderma sp. 'HSA12'
<400> 12
   gacaaagaag ccgaggacag 20
<210> 13
   <211> 20
   <212> DNA
   <213> Trichoderma sp. 'HSA12'
<400> 13
   atcaatagac ggggcatacg 20
<210> 14
   <211> 20
   <212> DNA
   <213> Trichoderma sp. 'HSA12'
<400> 14
   cgaaaagaga gccaaaaacg 20
<210> 15
   <211> 20
   <212> DNA
   <213> Trichoderma sp. 'HSA12'
<400> 15
   ggagaacgaa gcttgacctg 20
<210> 16
   <211> 20
   <212> DNA
   <213> Trichoderma sp. 'HSA12'
<400> 16
   tataccccgc ctcaacagtc 20
<210> 17
   <211> 20
   <212> DNA
   <213> Trichoderma sp. 'HSA12'
<400> 17
   tggtggtgtg tacgaaatgg 20
<210> 18
   <211> 20
   <212> DNA
   <213> Trichoderma sp. 'HSA12'
<400> 18
   ggcatcgtag cgaagtaagc 20
<210> 19
   <211> 20
   <212> DNA
   <213> Trichoderma sp. 'HSA12'
<400> 19
   tccaaaccct gactgaggtc 20
<210> 20
   <211> 20
   <212> DNA
   <213> Trichoderma sp. 'HSA12'
<400> 20
   agatgcagat cgtcgtgttg 20
<210> 21
   <211> 20
   <212> DNA
   <213> Trichoderma sp. 'HSA12'
<400> 21
   ctgcctctcc agaacactcc 20
<210> 22
   <211> 20
   <212> DNA
   <213> Trichoderma sp. 'HSA12'
<400> 22
   cattataagg ggccacaacg 20
<210> 23
   <211> 20
   <212> DNA
   <213> Trichoderma sp. 'HSA12'
<400> 23
   tacagcacga agacgctctc 20
<210> 24
   <211> 20
   <212> DNA
   <213> Trichoderma sp. 'HSA12'
<400> 24
   aacagcgacc aagcataacc 20
<210> 25
   <211> 20
   <212> DNA
   <213> Trichoderma sp. 'HSA12'
<400> 25
   ctgtcgagat tgctgctgag 20
<210> 26
   <211> 20
   <212> DNA
   <213> Trichoderma sp. 'HSA12'
<400> 26
   atgtactttt ccgcgtccag 20
<210> 27
   <211> 20
   <212> DNA
   <213> Trichoderma sp. 'HSA12'
<400> 27
   ttcaacagcg tcaacctcag 20
<210> 28
   <211> 20
   <212> DNA
   <213> Trichoderma sp. 'HSA12'
<400> 28
   ccggatttat tttggtggtg 20
<210> 29
   <211> 20
   <212> DNA
   <213> Trichoderma sp. 'HSA12'
<400> 29
   catttggggt gggtattctg 20
<210> 30
   <211> 20
   <212> DNA
   <213> Trichoderma sp. 'HSA12'
<400> 30
   attgtcaccg atggaggaag 20
<210> 31
   <211> 33
   <212> DNA
   <213> Trichoderma sp. 'HSA12'
<400> 31
   gaagaagaag aagaagaaga agaagaagaa gaa 33
<210> 32
   <211> 36
   <212> DNA
   <213> Trichoderma sp. 'HSA12'
<400> 32
   tcctcctcct cctcctcctc ctcctcctcc tcctcc 36
<210> 33
   <211> 33
   <212> DNA
   <213> Trichoderma sp. 'HSA12'
<400> 33
   catcatcatc atcatcatca tcatcatcat cat 33
<210> 34
   <211> 45
   <212> DNA
   <213> Trichoderma sp. 'HSA12'
<400> 34
   cttcttcttc ttcttcttct tcttcttctt cttcttcttc ttctt 45
<210> 35
   <211> 39
   <212> DNA
   <213> Trichoderma sp. 'HSA12'
<400> 35
   agtagtagta gtagtagtag tagtagtagt agtagtagt 39
<210> 36
   <211> 63
   <212> DNA
   <213> Trichoderma sp. 'HSA12'
<400> 36
   gaagtgaagg aagtgaagga agtgaaggaa gtgaaggaag tgaaggaagt gaaggaagtg 60
   aag 63
<210> 37
   <211> 40
   <212> DNA
   <213> Trichoderma sp. 'HSA12'
<400> 37
   tttgttttgt tttgttttgt tttgttttgt tttgttttgt 40
<210> 38
   <211> 28
   <212> DNA
   <213> Trichoderma sp. 'HSA12'
<400> 38
   gtgtgtgtgt gtgtgtgtgt gtgtgtgt 28
<210> 39
   <211> 24
   <212> DNA
   <213> Trichoderma sp. 'HSA12'
<400> 39
   tatatatata tatatatata tata 24
<210> 40
   <211> 25
   <212> DNA
   <213> Trichoderma sp. 'HSA12'
<400> 40
   tcaggtcagg tcaggtcagg tcagg 25
<210> 41
   <211> 30
   <212> DNA
   <213> Trichoderma sp. 'HSA12'
<400> 41
   cagcagcagc agcagcagca gcagcagcag 30
<210> 42
   <211> 18
   <212> DNA
   <213> Trichoderma sp. 'HSA12'
<400> 42
   aggaggagga ggaggagg 18
<210> 43
   <211> 21
   <212> DNA
   <213> Trichoderma sp. 'HSA12'
<400> 43
   tgctgctgct gctgctgctg c 21
<210> 44
   <211> 66
   <212> DNA
   <213> Trichoderma sp. 'HSA12'
<400> 44
   agtgccagtg ccagtgccag tgccagtgcc agtgccagtg ccagtgccag tgccagtgcc        60
   agtgcc                                                                   66
<210> 45
   <211> 34
   <212> DNA
   <213> Trichoderma sp. 'HSA12'
<400> 45
   atatatatat atatatatat atatatatat atat 34

## Beschreibung

Die Erfindung betrifft einen Pilzstamm der Gattung Trichoderma mit der Bezeichnung HSA12 sowie Zusammensetzungen, die diesen Pilzstamm oder dessen Sporen enthalten. Des Weiteren betrifft die Erfindung die Verwendung des Pilzstamms oder dessen Sporen in verschiedenen Verfahren, zum Beispiel in einem Verfahren zum Fördern und/oder zur Stabilisierung von Pflanzenwachstum und/oder zur Erhöhung der Erträge von Kulturpflanzen, die die Inokulation von Boden, Wurzeln und/oder oberirdischen Pflanzenteilen mit dem Pilzstamm oder Sporen oder mit diesen Pilzstamm oder dessen Sporen enthaltenden Zusammensetzungen umfassen, unter anderem für eine Erhöhung der Effizienz der Nährstoffaufnahme und für eine Verbesserung der Stresstoleranz von Kulturpflanzen. Des Weiteren betrifft die Erfindung die Verwendung des Pilzstamms oder dessen Sporen in einem Verfahren zur Verbesserung der Struktur und Gesundheit des Bodens oder zur Dekontamination oder Altlastensanierung eines Bodens oder eines Gewässers sowie in einem Verfahren zur Stabilisierung oder Neuansiedlung gefährdeter oder erwünschter Wildpflanzenpopulationen. Darüber hinaus betrifft die Erfindung einen Primerpaar-Satz für die Amplifikation von Mikrosatelliten-Loci des Genoms des Pilzstamms zur Entwicklung von molekularen Markern und zur Identifizierung des Pilzstamms sowie ein Verfahren zur Bestimmung des Pilzstamms.

Intensive Landwirtschaft verbraucht nichterneuerbare Ressourcen in Form von industriell hergestellten Mineraldüngern, zum Beispiel Phosphaten, und nutzt ökologisch fragwürdige chemische Pflanzenschutzmittel, die häufig in der Folge als Pestizidrückstände in Nahrungsmitteln zu finden sind. Gerade eine intensive Düngung mit Phosphat- und Stickstoffverbindungen eutrophiert durch Auswaschung Oberflächengewässer mit schwerwiegenden Folgen für Ökosysteme, wie Flüsse, Seen und Küsten, und kontaminiert außerdem das Grundwasser. Der Gesetzgeber, sowohl auf nationaler als auch auf der Ebene der Europäischen Union (EU), steuert mit Verschärfung der Grenzwerte gegen, zum Beispiel mit dem Nationalen Aktionsplan: "Nachhaltige Anwendung von Pflanzenschutzmitteln" und der Novellierung der EU-Düngeverordnung. Die Umsetzung dieser Richtlinien, die eine Reduktion der Pflanzenschutzmittel um 30 % und der Mineraldünger um bis zu 70 % vorsehen, geht mit massiven Ertragseinbußen der Landwirte einher, die ökonomisch kaum zu verkraften sind. Daher werden alternative Strategien zur Erhöhung der Nährstoffeffizienz und zur Stärkung der pflanzlichen Widerstandskraft gegen biotische und abiotische Stressfaktoren in der Landwirtschaft dringend benötigt.

Ein Lösungsweg ist der Einsatz von Bodenmikroorganismen. Diese haben zahlreiche unspezifische Eigenschaften, wie zum Beispiel die Verminderung der Nährstoffauswaschung oder Verbesserung der Bodenstruktur, die vielen, wenn nicht sogar allen, Bodenmikroorganismen zugeschrieben werden können. Es existieren jedoch auch hochspezifische Eigenschaften, die auf symbiotische Interaktionen mit Pflanzenwurzeln zurückzuführen sind und die von Spezies zu Spezies und sogar von Stamm zu Stamm innerhalb einer Spezies von Bodenmikroorganismen stark variieren können. Hierzu zählen unter anderem die Mobilisierung und Bereitstellung von Nährstoffen, wie zum Beispiel Phosphat, Stickstoff und Spurenelementen, aus dem Boden sowie die Induktion von Resistenz- und Stressabwehrgenen und damit einhergehend eine erhöhte Toleranz gegen biotische und abiotische Stressfaktoren.

Bisher ist in diesem Zusammenhang insbesondere der Einsatz von Pilzen der Gattung Trichoderma als Bodenmikroorganismen bekannt. Trichoderma ist eine größere Pilzgattung mit bisher 314 charakterisierenden Spezies. Die Gattung ist in der Biotechnologie weit verbreitet und wird zur Enzymproduktion für die Waschmittelindustrie und Laboranalytik eingesetzt. Das Angebot an Trichoderma-Produkten für Gartenbau und Landwirtschaft ist zumindest in Deutschland noch sehr übersichtlich. Die entsprechenden Produkte werden meist als sogenannte Bodenhilfsstoffe oder Pflanzenstärkungsmittel vertrieben, seltener als biologische Düngemittel. Es handelt sich um die Produkte Trichostar^{®}, Trichosan^{®}, Vitalin^{®}, "Promot", Trianum^{™}, "Triprof", "Mycorrmax", Biohealth^{™}, "T-Gro", "Tripol" und "AcTRIvator" sowie um einige Kombinationsprodukte, die zusätzlich Rhizobakterien enthalten. Bei den eingesetzten Trichoderma-Spezies zur Unterstützung der Pflanzenproduktion handelt es sich laut Herstellerinformation um verschiedene Stämme von Trichoderma harzianum, Trichoderma virens, Trichoderma asperellum, Trichoderma artroviride und Trichoderma koningii mit verschiedenen, meist niedrigen, Sporenkonzentrationen in den Produkten.

Verschiedene Stämme von Trichoderma werden in der WO 2010/091337 zur Förderung des Pflanzenwachstums und Steigerung des Ertrags, auch unter den Bedingungen abiotischen Stresses, untersucht.

Einige Stämme, wie zum Beispiel der im Produkt Trianum^{®} eingesetzte Trichoderma Harzianum-Stamm T22, können mineralisch gebundenes Phosphat lösen, haben aber keinen positiven Einfluss auf das Wurzelwachstum. Andere Hersteller, wie zum Beispiel die Partner Plant GmbH mit ihrem Produkt "Promot", versuchen, mehrere verschiedene Stämme mit unterschiedlichen Wirkungen auf Kulturpflanzen in einem Produkt zu kombinieren und hoffen so, beide positive Eigenschaften für Kulturpflanzen verfügbar zu machen. Dies gelingt jedoch zumeist nicht, da aufgrund der Konkurrenz um Nährstoffe und unterschiedlicher Fitness der Stämme in den vorgegebenen Bodenarten beide Stämme nicht gleichmäßig wachsen, sondern einer der Stämme aufgrund besserer Anpassung an die vorgegebenen Bedingungen schneller wächst und die Wurzel früher besiedelt. Zudem selektieren die Pflanzenwurzeln Pilzstämme in ihrer Rhizosphäre. Hat ein Stamm die Wurzel besiedelt und zum Beispiel lokale Resistenzmechanismen in der Wurzel ausgelöst, zum Beispiel die Verstärkung der pflanzlichen Zellwände durch Kallose-Einlagerung, können weitere Stämme nur verzögert oder gar nicht mehr in die Wurzel eindringen. Dadurch kommen deren positive Einflüsse auf den pflanzlichen Stoffwechsel auch nicht zum Tragen. Es kommt also nur zu einer effizienten Mehrfachbesiedlung, wenn zufällig Bedingungen vorliegen, unter denen die unterschiedlichen Stämme gleich gut wachsen und mehr oder minder gleichzeitig die Wurzel erreichen, bevor Abwehrmechanismen in der Wurzel ausgelöst und physiologisch umgesetzt worden sind. Dies betrifft zum Beispiel den wenige Stunden umfassenden Zeitraum von der Erkennung der Wurzelbesiedlung, der Signalerkennung durch die Pflanze, über die Transduktion des Signals in physiologische Reaktionen bis zur Einlagerung der Kallose in die Zellwände. Die gleichmäßige Ausprägung der verschiedenen Wirkungen unterschiedlicher Stämme ist also nur unter sehr seltenen Bedingungen erfolgreich.

Die der Erfindung zugrunde liegende Aufgabe besteht insbesondere darin, ein Isolat von Mikroorganismen bereitzustellen, das mehrere für Kulturpflanzen positive Wirkungen vereinigt.

Die Aufgabe der Erfindung wird gemäß Anspruch 1 gelöst durch einen Pilzstamm der Gattung Trichoderma mit der Bezeichnung HSA12, welcher am 12. Januar 2018 unter der Patenthinterlegungsbezeichnung Nummer DSM 32722 beim Leibniz-Institut DSMZ- Deutsche Sammlung von Mikroorganismen und Zellkulturen GmbH hinterlegt wurde, oder durch Sporen davon. Dieser Pilzstamm wird im Folgenden auch Trichoderma-Spezies-Stamm HSA12 oder kurz T. sp. HSA12 bezeichnet. Darüber hinaus stellt auch jede Zusammensetzung, die den genannten Trichoderma-Spezies-Stamm HSA12 oder Sporen davon umfasst, eine Lösung der Aufgabe der Erfindung dar. Vorteilhafte Weiterbildungen sind jeweils in den abhängigen Ansprüchen angegeben. So kann die Zusammensetzung neben dem Trichoderma-Spezies-Stamm HSA12 oder dessen Sporen ferner einen Träger umfassen. Darüber hinaus sind in der Zusammensetzung auch Kombinationen des Trichoderma-Spezies-Stamms HSA12 oder dessen Sporen mit anderen Mikroorganismen und/oder synthetischen oder biologischen Düngern und/oder Hilfsstoffen/ Adjuvantien und Trägermaterialien möglich. Die Kombination mit anderen Mikroorganismen betrifft vor allem gramnegative und/oder grampositive Prokaryoten wie Bakterien und Archaeen. Es können auch weitere Mikropilze oder aber Mikroprotisten, zum Beispiel Oomyceten mit positiven Eigenschaften für das Pflanzenwachstum zugegeben werden. Als synthetische oder biologische Düngerzusätze werden mineralische Nährstoffe wie Stickstoff, Phosphor, Kalium und Spurenelemente aber auch amorphes Silizium und als biologische Zusätze unter anderem Kompost- und Algenextrakte sowie Huminsäuren eingesetzt. Die zugesetzten Hilfsstoffe/Adjuvantien sind von der Applikationsform abhängig. Bei der Samenbeschichtung/Samenummantelung mit vitalem T. sp. HSA12 werden Haftmittel, Netzmittel und Stabilisatoren eingesetzt. Bei Flüssigpräparaten für die Blatt- und Bodenapplikation kommen Dispergiermittel/Emulgatoren, UV-Schutzpigmente, Konservierungsstoffe, Frostschutzmittel, Netzmittel und Schaumminderer zum Einsatz, und bei der Verwendung von Granulaten für die Bodenapplikation werden Haftmittel, Konservierungsstoffe und Stabilisatoren eingesetzt. Verwendete Trägerstoffe sind mineralischen Ursprungs und werden den Trockenpräparaten als Pulver beigemischt, zum Beispiel Kaolin, Bentonit, Ton oder Diatomeenerde.

Bei dem Pilzstamm HSA12 handelt es sich um einen Wurzelendophyten, der mit seinen Wirtspflanzen eine Endosymbiose eingeht, das heißt, die Pilzhyphen dringen in Wurzelzellen auch tiefer gelegener Gewebe ein. Der Pilz wurde dementsprechend aus der Wurzel einer Wildpflanze des Mitteldeutschen Trockengebietes isoliert, nicht aber aus dem Boden, und weist ein sehr weites Wirtsspektrum auf. Dies konnte durch Inokulationsversuche in den Wurzeln aller bisher untersuchten Kulturpflanzen nachgewiesen werden. Durch Isolation, Formulierung und darauf folgendem Einsatz des Pilzisolats in Topf- und Freilandexperimenten im Vergleich zu unbehandelten Kulturpflanzen konnte durch intensive Forschung gezeigt werden, dass HSA12 zahlreiche, für seine Wirtspflanzen positive Eigenschaften in sich vereinigt:
I eine wurzelendophytische Lebensweise, das heißt, dass HSA12 Pflanzenwurzeln besiedelt, ohne Schadsymptome auszulösen, was eine sogenannte Mykorrhiza, das heißt eine Form der Symbiose von Pilzen und Pflanzen, darstellt, bei der ein Pilz mit dem Feinwurzelsystem einer Pflanze in Kontakt ist, wobei der Nachweis über DNA/Polymerase-Kettenreaktion (PCR) erfolgt;
II eine Vergrößerung der Wurzeloberfläche, wobei die intrazellulären/wurzelendophytischen Pilzhyphen der Endosymbiose mit dem Bodenmycel verbunden sind, was eine höhere Trockenstresstoleranz zur Folge hat;
III eine Verbesserung der Rhizosphärenkompetenz, wobei HSA12 jederzeit im Wurzelbereich nachweisbar ist und von der Pflanze nicht durch Ausscheidung antifungaler Substanzen bekämpft wird;
IV eine Induktion des Wurzelwachstums und dadurch verbesserte Standfestigkeit und Trockenstresstoleranz der Wirtspflanzen, speziell bei Tomate und Mais;
V eine verbesserte Nährstoff- und Spurenelementeaufnahme ;
VI erhöhte Erträge gegenüber den unbehandelten Kontrollen, insbesondere bei Tomate und Mais;
VII eine erhöhte Aktivität der Mn-abhängigen Superoxid-Dismutase (SOD) in Stängeln und Blättern, woraus eine erhöhte Toleranz gegenüber oxidativem Stress durch reaktive Sauerstoffspezies, insbesondere in Mais, folgt;
VIII eine Erhöhung der Kältestresstoleranz in Mais, beispielsweise bei einer Wurzelraumtemperatur von 12 °C, was zu erhöhten Spiegeln von Polyphenol/Flavonoid/Prolin, welche Antistress-Metabolite sind, in Stängeln und Blättern und reduzierten Blattnekrosen führt;
IX eine Induktion eines frühen Blühzeitpunkts und Steigerung wertgebender Inhaltsstoffe im Erntegut von Kulturpflanzen.

Des Weiteren führt der Einsatz von Trichoderma sp. HSA12 offensichtlich zur
- Induktion von lokaler Resistenz in der Wurzel und systemischer Resistenz in der gesamten Pflanze und somit ein sogenanntes Priming des immanenten Immunsystems der Pflanzen;
- Mobilisierung von in Mineralien festgelegten Phosphaten, nachweisbar im Ca-Phosphat/Hydroxylapatit-Test,
- den Abbau schädlicher organischer Substanzen im Boden und in Gewässern, wie zum Beispiel von Phenolen, polyzyklischen Kohlenwasserstoffen und Toxinen.

Zusammenfassend kann festgestellt werden, dass der Trichoderma sp.-Stamm HSA12 zahlreiche positive Eigenschaften in sich vereinigt, die in dieser Kombination bisher einzigartig sind und von keinem anderen Stamm geboten werden. Daraus leiten sich zahlreiche Anwendungsmöglichkeiten für konventionelle Landwirtschaft, den Garten- und Gemüselandbau sowie den Biolandbau ab.

Aus den oben genannten Vorteilen ergeben sich Möglichkeiten der Verwendung des Pilzstamms beziehungsweise von dessen Sporen in Verfahren mit jeweils unterschiedlicher Zielstellung.

So betrifft ein Aspekt der Erfindung ein Verfahren zum Fördern und/oder zur Stabilisierung von Pflanzenwachstum und/oder zur Erhöhung der Erträge von Kulturpflanzen, umfassend die Inokulation des Bodens, der Wurzeln und/oder der oberirdischen Pflanzenteile mit dem genannten Trichoderma-Spezies-Stamm HSA12 oder Sporen davon oder einer Zusammensetzung, die diesen Pilzstamm oder Sporen davon umfasst.

Insbesondere führt die Inokulation des Bodens und/oder der Wurzeln mit dem des Pilzstamms Trichoderma sp. HSA12 oder dessen Sporen zur Mobilisierung mineralisch und/oder organisch gebundenen Phosphats zur Verbesserung der Kulturpflanzenernährung und Einsparung von Mineraldüngern. Die Anwendung des Pilzstamms Trichoderma sp. Stamm HSA12 oder dessen Sporen ermöglicht nämlich die Ausnutzung der natürlichen Boden-Phosphatreserven, zu denen Pflanzenwurzeln keinen Zugang haben, weil sie in Mineralien festgelegtes Phosphat nicht lösen und aufnehmen können. Zusätzlich kann aufgrund der Phytase-Aktivität auch eine Nutzung organisch gebundenen Phosphats aus Pflanzenresten erfolgen, welches Pflanzen ebenfalls nicht aufnehmen können. Letzteres lässt eine höhere Effizienz bei Gründüngung erwarten. Durch diese beiden Eigenschaften können auch bei einer massiven Reduktion der Mineraldünger die Erträge weitgehend stabil gehalten werden.

Darüber hinaus wird durch die Inokulation des Bodens und/oder der Wurzeln mit dem Pilzstamm Trichoderma sp. HSA12 oder dessen Sporen auch das Wurzelwachstum der Kulturpflanzen gefördert, einhergehend mit einer verbesserten Nährstoff-, Spurenelement- und Wasseraufnahme der Kulturpflanzen. Dadurch ist eine weitere Steigerung der Erträge möglich. Durch das Wurzelwachstum wird zudem die Standfestigkeit und Trockenstresstoleranz vieler Kulturpflanzen verbessert, was bei Extremwetterereignissen, wie Starkwind, Starkregen oder Dürre, von Vorteil sein kann, um den Herausforderungen des Klimawandels zu begegnen.

Durch die Eigenschaften des Pilzstamms Trichoderma sp. HSA12, wie die Förderung der Trockenstresstoleranz, der Kältestresstoleranz und der oxidativen Stresstoleranz von Kulturpflanzen, insbesondere durch Förderung der Expression und Aktivität von Antistressmetaboliten in den Kulturpflanzen, ist es vorteilhafterweise möglich, dass das Pflanzenwachstum unter abiotischem Stress stattfindet. Dieser abiotische Stress kann in Form von Trockenstress, Kältestress und/oder oxydativem Stress vorliegen. Der abiotische Stress kann beispielsweise im Ergebnis eines, bezogen auf die jeweilige Region, nach vorne oder nach hinten verschobenen Aussaat- oder Auspflanzungstermins für die Kulturpflanze vorliegen.

Insbesondere die größere Toleranz der behandelten Kulturpflanzen gegenüber Kältestress ist eine Eigenschaft, die im Zusammenhang mit anderen Pilzstämmen bisher nicht beobachtet werden konnte. So ergeben sich bezüglich einiger Pflanzenarten, wie Soja und Mais, neue Möglichkeiten, für den Anbau kühlere Breiten zu erschließen. Bei Mais muss beispielsweise in Mitteleuropa bisher der späte Aussaattermin in der zweiten Maiwoche eingehalten werden. Dieser Termin kann durch den Einsatz von Trichoderma sp. HSA12 und durch die damit verbundene Erhöhung der Kältestresstoleranz vorgezogen werden. Auf diese Weise können höhere Erträge auch in kühleren Breiten erzielt werden.

Die Behandlung der Kulturpflanze mit dem Pilzstamm Trichoderma sp. HSA12 oder dessen Sporen führt darüber hinaus auch zur Induktion beziehungsweise Stimulation des pflanzlichen Immunsystems und zur Aktivierung von Resistenzgenen in Kulturpflanzen. Die Behandlung führt auch zu einer Erhöhung der allgemeinen Widerstandsfähigkeit (Resilience) von Kulturpflanzen.

Die Erhöhung der Erträge steht auch im Zusammenhang mit der durch die oben genannte Inokulation ebenfalls beobachtete Induktion eines frühen Blühzeitpunkts bei Kulturpflanzen. Ebenso konnte die Steigerung wertgebender Inhaltsstoffe im Erntegut von Kulturpflanzen, wie zum Beispiel Tomaten, beobachtet werden.

Ein anderer Aspekt der Erfindung betrifft ein Verfahren zur Verbesserung der Struktur und Gesundheit des Bodens oder zur Dekontamination oder Altlastensanierung eines toxische organische Substanzen enthaltenden Bodens oder eines Gewässers, umfassend eine Inokulation des Bodens oder des Gewässers mit dem Pilzstamm Trichoderma sp. HSA12 oder Sporen oder mit dem Pilzstamm oder dessen Sporen enthaltenden Zusammensetzungen und Kultivieren des Trichoderma-Spezies-Stamms HSA12 und gegebenenfalls weiterer Stämme der Zusammensetzung im Boden oder im Gewässer.

Ein zusätzlicher Aspekt der Erfindung betrifft ein Verfahren zur Stabilisierung oder Neuansiedlung gefährdeter oder erwünschter Wildpflanzenpopulationen, umfassend eine Inokulation von Boden, Wurzeln und/oder oberirdischen Pflanzenteilen der Wildpflanzen mit dem Pilzstamm Trichoderma sp. HSA12 oder Sporen oder mit dem Pilzstamm oder dessen Sporen enthaltenden Zusammensetzungen.

Die Verwendung des Pilzes muss in allen oben genannten Verfahren als lebendige Kultur in Form von vitalen Sporen oder vermehrungsfähigen Hyphen erfolgen, da der Pilzstamm seine Wirkung nur als lebender Organismus entfalten kann.

Trichoderma gehört zur Abteilung der Ascomycota (Schlauchpilze), die sich besonders einfach auf Nährmedien kultivieren lassen und sich dadurch auszeichnen, währenddessen große Mengen an vegetativen Sporen zu bilden. Daher steht einer Massenproduktion für die Landwirtschaft nichts im Wege.

Eine besonders bevorzugte Ausführungsform betrifft eine Zusammensetzung und deren Verwendung in den oben beschriebenen Verfahren, wobei diese Zusammensetzung neben dem Pilzstamm Trichoderma sp. HSA12 als weiteren Mikroorganismus ein Rhizobakterium umfasst, mit dem Trichoderma sp. HSA12 synergistisch interagiert. Dies konnte zum Beispiel für den Stamm Bacillus amyloliquefaciens FZB42 beobachtet werden, welcher als Produkt RhizoVital von der Firma AbiTEP, Berlin hergestellt und vertrieben wird. Werden Pflanzenwurzeln, zum Beispiel Mais und Tomate, einzeln mit HSA12 beziehungsweise FZB42 inokuliert und das Ergebnis mit Wurzeln verglichen, die mit HSA12 und FZB42 inokuliert wurden, ist ein klarer Synergismus zu erkennen. Die Kombination erzielt stets bessere Ergebnisse als die Einzelinokulationen. Dies zeigte sich im Fall von Mais und Tomate durch ein kräftigeres Wurzelwachstum, eine frühere Blüte und in Folge dessen durch einen signifikant höheren Ertrag im Vergleich zu den mit nur einer Kultur inokulierten Pflanzen und zu den unbehandelten Kontrollen.

Durch die Verfügbarkeit der kompletten Genomsequenz, insgesamt 39,69 Mb Megabasenpaare einschließlich Mitochondrien DNA-Sequenz, kann der Stamm jeder Zeit in der Umwelt oder in etwaigen Produkten identifiziert werden. In diesem Zusammenhang ist als weiterer Aspekt der Erfindung ein Primerpaar-Satz für die Amplifikation von Mikrosatelliten-Loci des Genoms des Pilzstamms zur Bestimmung von molekularen Markern und zur Identifizierung des Pilzstamms zu nennen. Dabei werden die jeweiligen Primerpaare aus den Primern mit den weiter unten aufgeführten Primersequenzen SEQ-ID-Nr. 1 und 2; 3 und 4; 5 und 6; 7 und 8; 9 und 10; 11 und 12; 13 und 14; 15 und 16; 17 und 18; 19 und 20; 21 und 22; 23 und 24; 25 und 26; 27 und 28; 29 und 30 gebildet. Mittels des Primerpaar-Satzes kann ein Marker-Satz zur Bestimmung des Pilzstamms Trichoderma sp. HSA12 amplifiziert werden, umfassend die weiter unten aufgeführten Sequenzen mit den SEQ-ID-Nr. 31 bis 45.

Ein entsprechendes Verfahren zur Bestimmung des Pilzstamms nach Anspruch umfasst die Verfahrensschritte
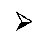 Gewinnung der DNA aus einer Probe, die den Pilz enthält, zum Beispiel Wurzeln, Boden oder Kulturen,
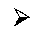 Amplifikation von Genomabschnitten als Mikrosatelliten-Loci zur Bestimmung von molekularen Markern mittels der Primerpaare des genannten Primerpaarsatzes und der Polymerase Kettenreaktion (PCR),
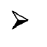 Bestimmung der Fragmentlänge der jeweiligen Genomabschnitte, angegeben in Basenpaaren (bp), und/oder der Art sich wiederholenden Sequenzen und/oder der jeweiligen Anzahl der Sequenzwiederholungen (Repeat-Anzahl) in den Genomabschnitten und Vergleich mit den entsprechenden Eigenschaften des Pilzstamms Trichoderma sp. HSA12.
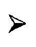 Weitere Einzelheiten, Merkmale und Vorteile von Ausgestaltungen der Erfindung ergeben sich aus der nachfolgenden Beschreibung von Ausführungsbeispielen mit Bezugnahme auf die zugehörigen Zeichnungen.

Es zeigen:
- **Figur 1:**: ein Säulendiagramm mit einem Vergleich der Erträge von Zwergtomaten mit und ohne T. sp. HSA12-Behandlung,
- **Figuren 2A** - **C:**: die Ergebnisse von Topfexperimenten mit unterschiedlich behandelten Maispflanzen anhand von Säulendiagrammen,
- **Figur 2D:**: die Ergebnisse von Topfexperimenten mit unterschiedlich behandelten Maispflanzen anhand einer fotografischen Abbildung,
- **Figur 3:**: eine fotografische Abbildung, die Tomatenpflanzen nach verschiedenen Kultivierungsbedingungen zum Zeitpunkt der abschließenden Ernte einander gegenübergestellt,
- **Figur 4:**: Säulendiagramm, das die jeweilige Anzahl der geschädigten Blätter bei der Kultivierung von Mais unter verschiedenen Bedingungen einander gegenüberstellt,
- **Figuren 5A** - **B:**: Säulendiagramme, aus denen eine Induktion eines frühen Blühzeitpunkts und die Steigerung wertgebender Inhaltsstoffe im Erntegut von Tomaten durch die frühzeitige Blüte bei Zusatz von Trichoderma sp. HSA12 hervorgeht,
- **Figuren 6A-B:**: die Ergebnisse von Topfexperimenten mit Tomatenpflanzen vom Typ. Harzfeuer anhand von Säulendiagrammen,
- **Figur 6C:**: die Ergebnisse von Topfexperimenten mit Tomatenpflanzen vom Typ. Harzfeuer anhand einer fotografischen Abbildung.

Die **Tabelle 1** zeigt anhand einer Gegenüberstellung mit einem bekannten Bioeffektor-Produkt eine Induktion des Wurzelwachstums und dadurch verbesserte Standfestigkeit und Trockenstresstoleranz der Wirtspflanzen, speziell bei Mais, durch die Inokulation mit dem Pilzstamm HSA12 der Gattung Trichoderma, im Folgenden Trichoderma spezies HSA12 oder kurz T. sp. HSA12 bezeichnet, wobei die Steigerung 130 bzw. 190 % beträgt.

**Tabelle 1**

| | Spross-Biomasse | Wurzellänge | Phosphorgehalt des Sprosses |
|---|---|---|---|
| NO₃⁻ + lösliches Phosphat | 100 % (111g) | 100 % (4900 cm) | 100 % (24 mg / Pflanze) |
| NH₄ RP | 59 | 126 | 73 |
| NH₄RP Trianum^{™} | 75b | 117 | 84 b |
| NH₄RP T. sp. HSA12 | 86 ab | 190 b | 78 |
| RP = Rohphosphat | | | |
| a = nicht signifikant größer als bei Zuführung von Phosphat; | | | |
| b = unterscheidet sich signifikant vom Vergleichswert des NH4 RP | | | |

Das induzierte Wachstum in Mais hängt von der Form der Stickstoff- und Phosphorversorgung ab. Die Versuche wurden auf einem phosphorarmen Lehmboden mit 20 mg pflanzenverfügbarem Phosphor pro kg Boden durchgeführt. Die Menge des Phosphors wurde mittels Calcium-Acetat-Lactat-Auszug, kurz CAL-Auszug, als allgemein bekanntes Verfahren zur Extraktion von pflanzenverfügbarem Phosphor bestimmt. Die Abkürzung RP steht für Rohphosphat. T. sp. HSA12 induziert ein stärkeres Wachstum als das Bioeffektor-Produkt Trianum^{™}. Im Gegensatz zum Trianum^{™} hat der T. sp. HSA12 keinen Effekt auf den Anteil des Phosphors im Spross, induziert aber einen starken Anstieg des Wurzelwachstums (190 %), der, wie die Gegenüberstellung der jeweiligen Wurzellängen zeigt, deutlich stärker ist als bei der Behandlung mit Trianum^{™}. Daraus folgt, dass T. sp. HSA12 die Entwicklung von Mais durch Steigerung des Wurzelwachstums fördert.

Die **Tabelle 2** zeigt den jeweiligen Gehalt an Stickstoff N, Phosphor P, Kalium K und Mangan Mn im Spross von Mais, wobei aus der Tabelle 2 deutlich wird, wie dieser Gehalt durch die Inokulation mit T. sp. HSA12 und - damit verbunden - durch die Art der Stickstoff- und Phosphorversorgung beeinflusst wird. Der stärker wachstumsfördernde Effekt von T. sp. HSA12 auf die Pflanze im Vergleich zum Produkt Trianum^{™} kann mit dem verbesserten Zugang zu mehreren Nährstoffen, nämlich Stickstoff N, Kalium K und Mangan, erklärt werden, herbeigeführt durch ein verstärktes Wurzelwachstum. So ist insbesondere im Falle von Stickstoff N und Mangan Mn eine verbesserte Nährstoff- und Spurenelementeaufnahme bei Mais zu verzeichnen.

**Tabelle 2**

| | N (mg pro Pflanze) | P (mg pro Pflanze) | K (mg pro Pflanze) | Mn (mg pro Pflanze) |
|---|---|---|---|---|
| NO₃⁻+ lösliches Phosphat | 100 % (289) | 100 % (24) | 100 % (34) | 100 % (0,26) |
| NH₄ RP | 93 a | 73 | 70 | 94 a |
| NH₄RP Trianum^{™} | 109 a | 84 b | 85 b | 111 a |
| NH₄RP T. sp. HSA12 | 122 ab | 78 | 87 ab | 128 ab |
| RP = Rohphosphat | | | | |
| a = nicht signifikant größer als bei Zuführung von Phosphat; | | | | |
| b = unterscheidet sich signifikant vom Vergleichswert des NH₄ RP | | | | |

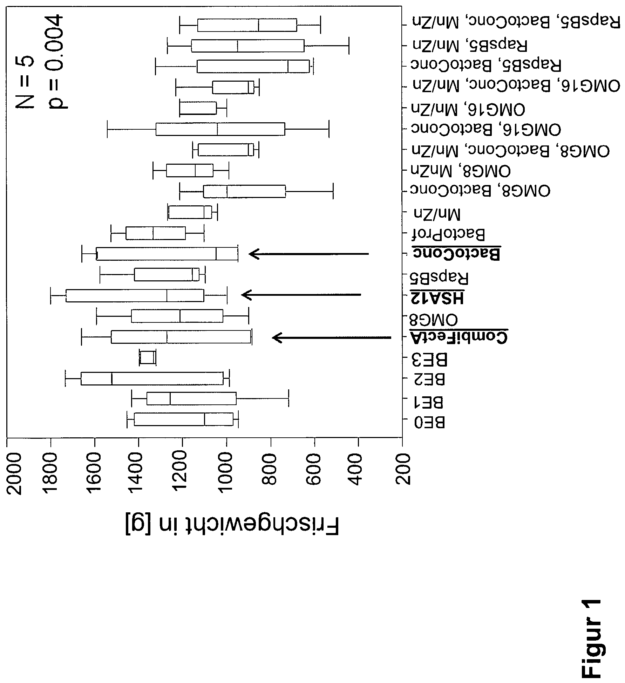

Die **Figur 1** zeigt anhand eine Säulendiagramms den Einfluss von T. sp. HSA12 auf den Ertrag von Zwergtomaten (Tomato cv. DwarfTom) gegenüber den unbehandelten Kontrollen bei Zwergtomaten. Die Angaben des Ertrages erfolgen als Frischgewicht der Tomatenfrüchte. Die Versuche wurden in Löss-Schwarzerdeboden ohne Phosphordüngung und einem Wert von pH 7, 2 durchgeführt.

Beim Wert N handelt es sich um die Stichprobengröße pro Prüfglied. Wenn N = 5, so entspricht das einer Stichprobengröße von 5 Pflanzen pro Behandlung. Der Wert, der dem kleinen Buchstaben p im Diagramm zugeordnet ist, ist eine Angabe für das Signifikanzniveau. Ein Wert p = 0.004, der deutlich kleiner ist als 0,05, zeigt eine sehr starke Signifikanz an.

Die Abkürzung BE steht für einen "BioEffektor" für die mikrobielle Wachstumsförderung. BE0 ist demgegenüber die Kurzbezeichnung für eine unbehandelte Kontrolle. Bei BE1 handelt es sich um Trianum^{®}P, ein kommerzielles Produkt der Firma Koppert Ltd. aus den Niederlanden, welches einen Trichoderma-Pilzstamm mit der Bezeichnung T22 beinhaltet. Bei BE2 handelt es sich Proradix, ein kommerzielles Bakterienprodukt (Pseudomonas) der Firma Sourcon-Padena GmbH, Tübingen. Bei BE3 handelt es sich um den Stamm FZB42 der Bakteriengattung Bacillus amyloliquefaciens, welcher auch als Produkt RhizoVital^{™} von der Firma AbiTEP Berlin hergestellt und vertrieben wird. CombifectA besteht aus einer Kombination von T. sp. HSA12 in Kombination mit 5 Bazillen BactoConc.

OMG8, RapB5 und OMG16 sind Abkürzungen für andere Trichodermastämme der Hochschule Anhalt als T. sp. HSA12. BactoConc ist ein Bakterienprodukt aus fünf verschiedenen Bacillusstämmen, BactoProf ist ein Bakterienprodukt aus verschiedenen Bakterien und Hilfsstoffen. Beides sind Produkte der Bactiva GmbH, Straelen. Zink und Mangan (Mn/Zn) werden in einigen Proben als Spurenelemente eingesetzt. Des Weiteren werden Mischungen der oben aufgeführten Komponenten verwendet. Für alle Proben gilt die Angabe P0, das heißt, es erfolgt keine zusätzliche Phosphatdüngung.

Bei Inkubation mit T. sp. HSA12 wurde ein Frischgewicht von > 1700 g ermittelt. Im Falle der BE2 waren es 1650 mg, bei BactoConc 1650 g, bei CombifectA > 1500 g. Gegenüber den unbehandelten Kontrollen sind erhöhte Erträge an Tomaten zu verzeichnen.

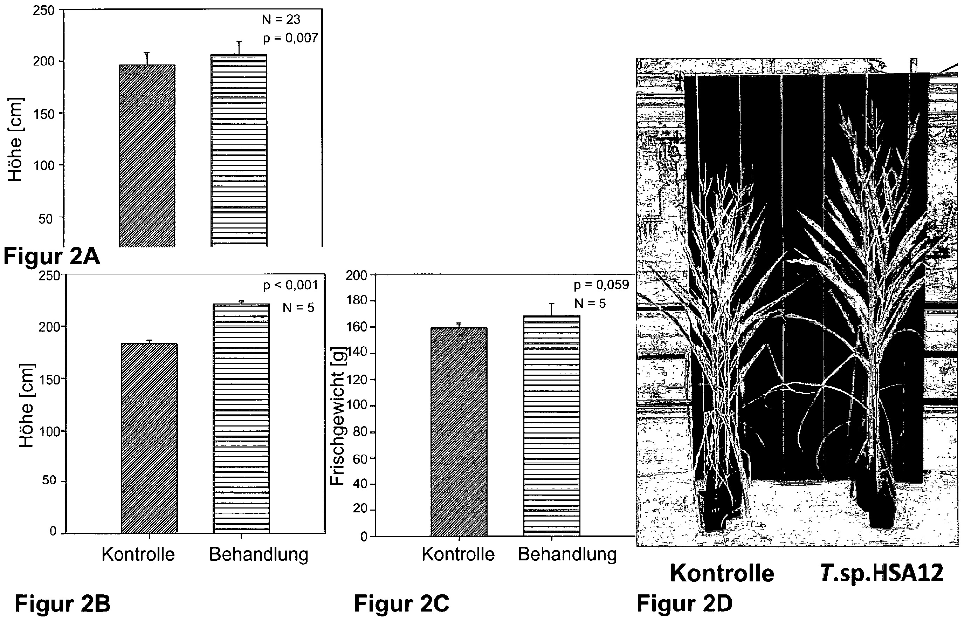

Die **Figuren 2A** bis D

zeigen die Ergebnisse eines Wachtumstests in Form von Topfexperimenten mit Mais. In diesen Experimenten wird, wie die Säulendiagramme in den **Figuren 2A** bis C

sowie die fotografische **Figur 2D** zeigen, der Einfluss von Trichoderma sp. HSA12 auf die Höhe in cm und das Frischgewicht in Gramm der oberirdischen Teile der Maispflanze gegenüber den unbehandelten Kontrollen deutlich. Der Wert für N gibt die Stichprobengröße an, die 5 beziehungsweise 23 Pflanzen beträgt, während der Wert für p eine Angabe für das Signifikanzniveau ist.

Die Ergebnisse belegen den signifkanten wachstumsfördernden Einfluss von Trichoderma sp. HSA12.

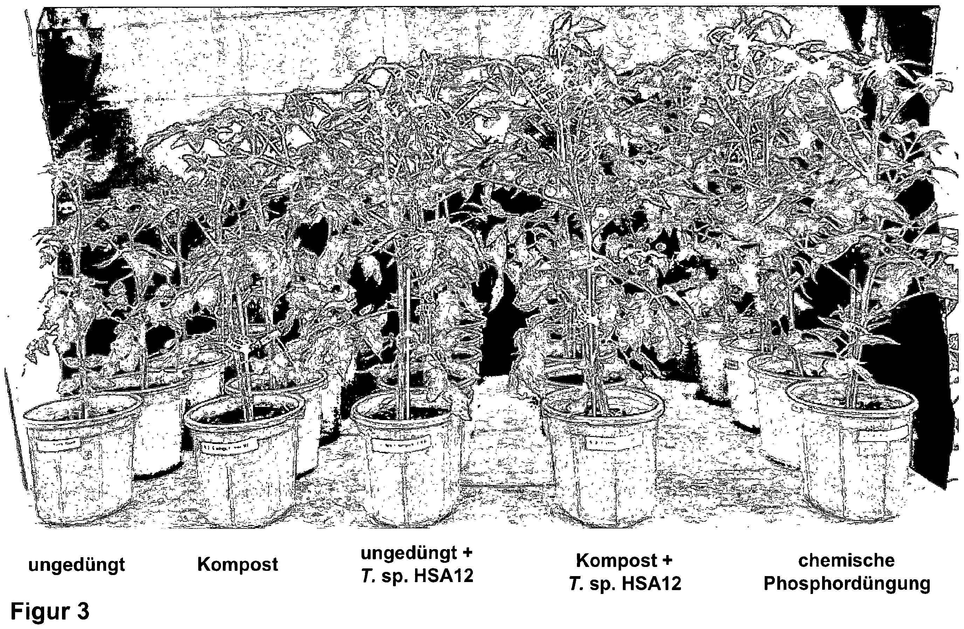

Die **Figur 3** zeigt eine fotografische Abbildung, die Tomatenpflanzen nach verschiedenen Kultivierungsbedingungen zum Zeitpunkt der abschließenden Ernte einander gegenübergestellt. So werden ungedüngte Pflanzen und in Komposterde gedüngte Pflanzen jeweils mit und ohne Trichoderma sp. HSA12-Inokulation kultiviert. Das Trockengewicht der ungedüngten Tomatenpflanze ohne T. sp. HSA12-Inokulation betrug 32,6 g, das Trockengewicht der ungedüngten Tomatenpflanze bei T. sp. HSA12-Inokulation betrug 49 g. Die Tomatenpflanze, die ohne T. sp. HSA12-Inokulation auf Komposterde gewachsen war, hatte ein Trockengewicht von 46,2 g, die Tomatenpflanze auf Komposterde mit T. sp. HSA12-Inokulation hatte ein Trockengewicht von 53 g. Im Vergleich dazu hatte eine Pflanze, die mit gelöstem Phosphat (Triple-Superphosphat) gedüngt wurde, ein Trockengewicht von 95,2 g. Letztere dient als Positivkontrolle dafür, was nur mit aufwändiger und umweltbelastender chemischer Düngung erreichbar ist.

Aus der **Tabelle 3** ist die Änderung der Abwehrreaktionen von Mais im Vergleich zu Topfexperimenten mit Mais in Nitrat (NO₃)-haltigem Boden zu entnehmen, wobei die Angaben in Prozent erfolgen. Die Ergebnisse zeigen, dass die Zugabe von Ammonium (NH₄⁺) Blattschäden reduziert und schützende Verbindungen, wie Prolin, Silizium (Si) und Superoxid-Dismutase (SOD), aktiviert. Die Zugabe von T. sp. HSA12 bringt zusätzliche Abwehrverbindungen hervor, wie Ascorbatperoxidase (APX), Antioxidantien, Wurzel-SOD und Wurzel-Prolin, was mit einer zusätzlichen Reduktion von Blattschäden verbunden ist. Zum Vergleich werden dem Boden beziehungsweise den Blättern in weiteren Kontrollen Zink und Mangan, die für den Biolandbau nicht zugelassen sind, Silizium oder "AlgaVyt", ein sehr mineralstoffreiches Algenprodukt, oder Superfifty^{®}, ein Seetangextrakt zur Verwendung als Wachstumsstimulator bei Pflanzen, oder wässrige Auszüge aus Kompost, so genannter Komposttee, zugesetzt. Aus der erhöhten Aktivität der Mn-abhängigen Superoxid-Dismutase (SOD) in Stängeln und Blättern folgt eine erhöhte Toleranz gegenüber oxidativem Stress durch reaktive Sauerstoffspezies im Mais.

**Tabelle 3**

| | | **Bodenbehandlung** | | | **Blattbehandlung** | | |
|---|---|---|---|---|---|---|---|
| **Abwehrreaktion [% Änderung im Vergleich zur NO₃- Kontrolle]** | NH₄⁺ allein | HSA12 | Zn/Mn | Si | AlgaVyt Zn/Mn | Superfifty^{®} | Komposttee |
| Wurzellänqe | k.A. | k.A. | k.A. | + 44 | k.A. | k.A. | k.A. |

| | | **Bodenbehandlung** | | | **Blattbehandlung** | | |
|---|---|---|---|---|---|---|---|
| Oxidativer Stress | | | | | | | |
| Blattschäden | - 17 | - 42 | -41 | - 33 | - 44 | - 34 | - 45 |
| SOD (Shoot) | + 41 | + 26 | + 15 | + 51 | + 44 | + 60 | + 51 |
| SOD (Root) | k.A. | + 89 | + 95 | + 110 | k.A. | k.A. | k.A. |
| APX (Shoot) | k.A. | + 47 | + 62 | + 54 | + 54 | + 59 | + 53 |
| Antioxidantien | + 19 | + 86 | + 79 | + 75 | + 76 | + 76 | + 76 |
| Phenole | k.A. | + 157 | + 119 | + 109 | + 96 | + 84 | + 96 |
| Schützende qelöste Stoffe | | | | | | | |
| Prolin (Spross) | + 275 | + 227 | + 333 | + 233 | + 120 | + 87 | + 35 |
| Prolin (Wurzeln) | k.A. | + 60 | + 96 | + 78 | + 180 | + 120 | + 80 |
| Nährstoffniveau | | | | | | | |
| Zink | + 33 | + 16 | + 105 | + 34 | + 56 | + 56 | + 56 |
| Si | + 43 | + 43 | + 43 | + 50 | + 40 | + 53 | + 37 |
| | k.A. = keine Angabe | | | | | | |

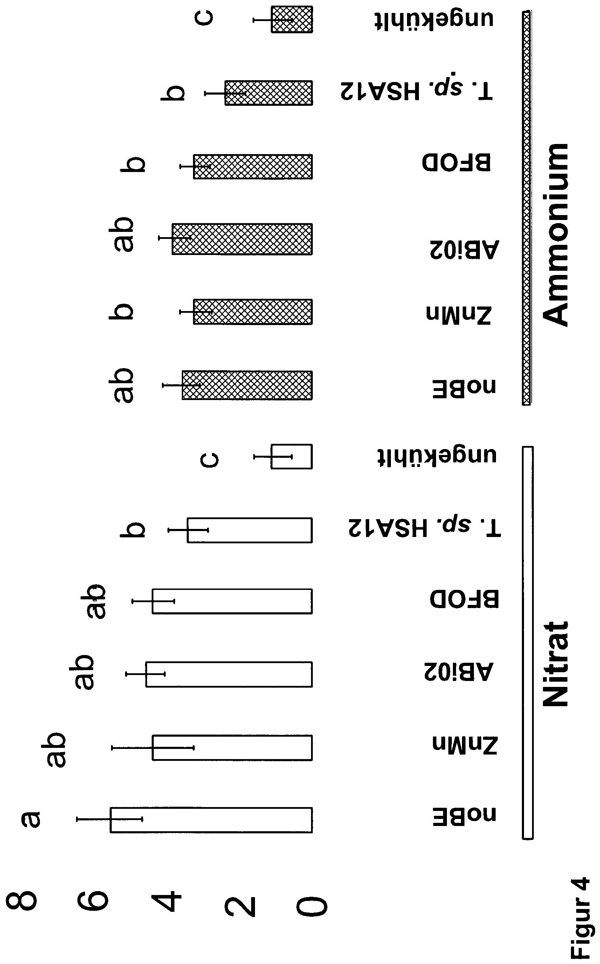

Die **Figur 4** zeigt die Ausbildung von Blattnekrosen bei Kältestress in Mais bei einer Wurzeltemperatur von 12°C. In einem Säulendiagramm werden die jeweilige Anzahl der geschädigten Blätter bei der Kultivierung von Mais unter verschiedenen Bedingungen einander gegenübergestellt. Die Pflanzen wurden entweder mit Nitrat (NO₃⁻) oder mit Ammonium (NH₄⁺) gedüngt. Darüber hinaus erfolgte in der unbehandelten, gekühlten Kontrolle noBE kein weiterer Zusatz von Spurenelementen oder Kulturen. Eine weitere Kontrolle erfolgte unter ungekühlten Bedingungen, das heißt bei einer Wurzeltemperatur von 18 °C. Die anderen Vergleichskultivierungen zur Kultivierung nach T. sp. HSA12-Inokulation erfolgten entweder unter Zusatz der Spurenelemente Zink und Mangan (Zn, Mn) oder Abi02, einem Bacilluspräparat der Firma ABITEP GmbH, oder BFOD - Penicillium (Pilz), einem Präparat der Firma Bayer Crop Science.

Nach Beginn der Kälteperiode von 14 Tagen mit Temperaturen von 12 °C setzt eine schnelle Entwicklung von oxidativen Blatt-Schädigungen ein, nämlich Nekrose, Chlorose und Anthocyanbildung. Der Blattschaden sinkt in folgender Reihenfolge:
Abi 02 + ZnMn > ZnMn > BFOD + ZnMn > T. sp. HSA12 > ungekühlte Kontrolle.

Die Wurzelraumtemperatur der gekühlten Pflanzen beträgt 12 °C. Grundsätzlich sind die mit Ammonium behandelten Pflanzen weniger geschädigt als die mit Nitrat behandelten Pflanzen. Weniger Blattschäden als die mit T. sp. HSA12 behandelten Pflanzen weisen nur noch die ungekühlten Pflanzen auf. Mit anderen Worten: Die ungekühlte Kontrolle hat natürlich die wenigsten Nekrosen, aber die zweitbesten Ergebnisse erzielt die Kultivierung nach T. sp. HSA12-Inokulation, die den Mais sehr gut vor Kälteschäden schützt. Bei Ammoniumdüngung ist der Schutz besser als bei der Nitratdüngung, wie die deutlich kleineren Säulen anzeigen.

In Folge der Inokulation mit T. sp. HSA12 kommt es zu einer Erhöhung der Kältestresstoleranz in Mais, was zu erhöhten Spiegeln von Polyphenol/Flavonoid/Prolin als Antistress-Metabolite in Stängeln und Blättern und reduzierten Blattnekrosen führt.

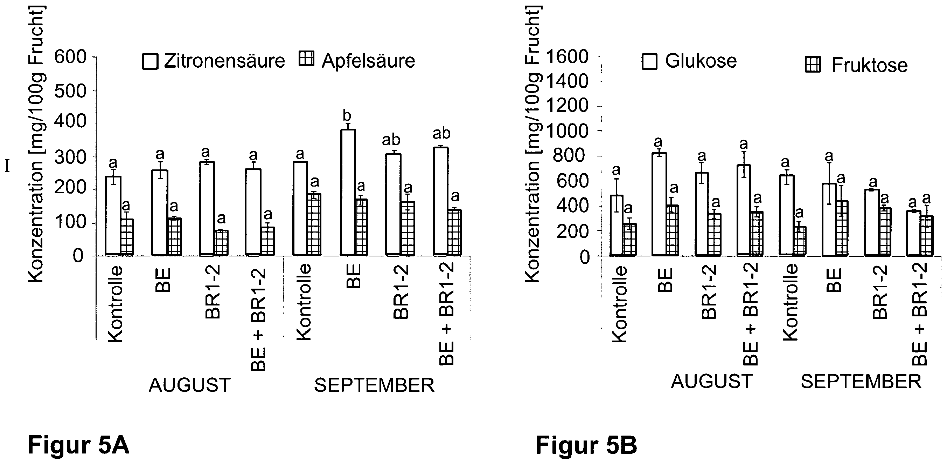

Die **Figuren 5A und 5B** zeigen anhand von Säulendiagrammen eine Induktion eines frühen Blühzeitpunkts und die Steigerung wertgebender Inhaltsstoffe im Erntegut von Tomaten der Sorte "MOBIL" aus Ungarn durch die frühzeitige Blüte bei Zusatz von Trichoderma sp. HSA12. Die Buchstaben über den Säulen markieren die Signifikanz, das heißt, überall wo gleiche Buchstaben erscheinen, bestehen keine signifikanten Unterschiede und das Signifikanzniveau ist mindestens p < 0.05.

Dabei sind in der **Figur 5A** die Konzentrationen von Zitronensäure und Maleinsäure (Apfelsäure), in der **Figur 5B** die Konzentrationen von Glukose und Fruktose unter jeweils vier verschiedenen Bedingungen einmal im August und einmal im September dargestellt. Es werden jeweils eine Kontrolle, eine Probe mit Zusatz von Trichoderma sp. HSA12, abgekürzt mit BE, eine Probe mit zwei bakteriellen Zusätzen, abgekürzt mit BR1 und BR2, und eine Probe mit einem kombinierten Zusatz von Trichoderma sp. HSA12 und einem der bakteriellen Zusätze, BR2, in den Säulendiagrammen gegenübergestellt.

Durch die Induktion eines früheren Blühzeitpunktes kommt es zur Steigerung wertgebender Inhaltsstoffe in Kulturpflanzen, im gezeigten Beispiel der Tomate. Während der Gehalt an Zitronensäure im August bei der Kontrolle bei 220 mg pro 100 g Frucht lag, lag er bei der Pflanze, die mit Trichoderma sp. HSA12 behandelt wurde, mit 250 mg pro 100 g Frucht diesem Zeitpunkt bereits deutlich höher. Im September war dieser Unterschied mit 380 mg Zitronensäure pro 100 g Frucht von der mit Trichoderma sp. HSA12 behandelten Pflanze gegenüber der Kontrolle mit nur 280 mg Zitronensäure pro 100 g Frucht noch größer.

Der Gehalt an Maleinsäure war in den Früchten nach der Behandlung mit Trichoderma sp. HSA im August etwa gleich und im September etwas geringer als in der Kontrolle.

Im August war in Folge des früheren Blühzeitpunktes der Gehalt an Glukose in Früchten der mit Trichoderma sp. HSA12 behandelten Pflanzen mit 850 mg pro 100 g Frucht wesentlich höher als in der entsprechenden Kontrolle, wobei der Gehalt an Glukose in der Kontrolle bei 480 mg pro 100 g Frucht lag. Im September war der Gehalt an Glukose in Früchten der mit Trichoderma sp. HSA12 behandelten Pflanzen mit 560 mg pro 100 g Frucht etwas geringer als in der entsprechenden Kontrolle, in der ein Wert von 620 mg pro 100 g Frucht vorlag.

Sowohl im August auch auch im September waren die Werte für die Fruktose in den Früchten der mit Trichoderma sp. HSA behandelten Pflanzen wesentlich höher als in der Kontrolle. Der Gehalt an Fruktose der mit Trichoderma sp. HSA behandelten Pflanzen betrug im August 400 mg pro 100 g Frucht und im September 420 mg pro 100 g Frucht, während die Kontrollwerte bei 260 beziehungsweise 220 mg pro 100 kg Frucht lagen.

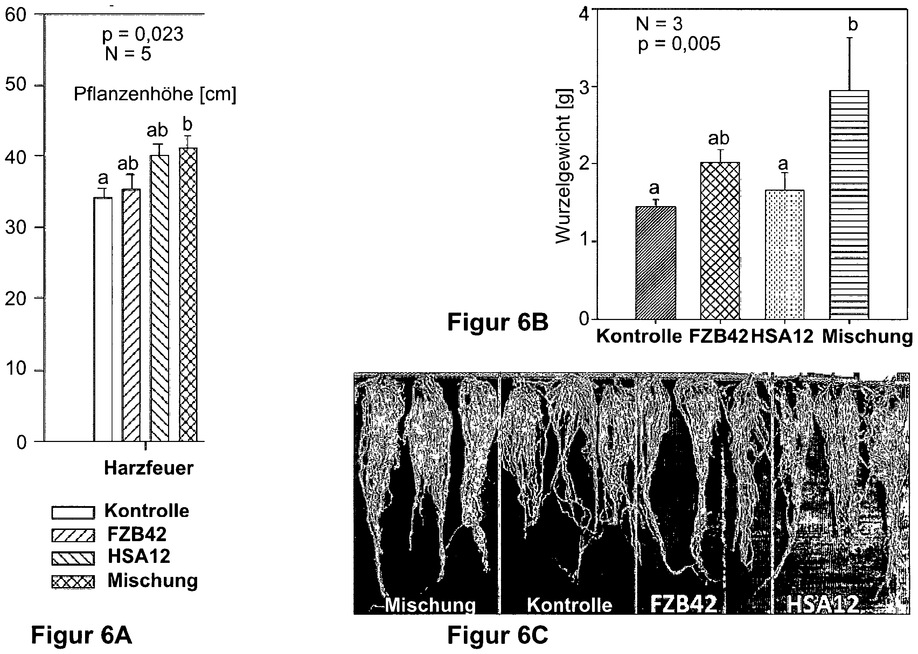

Die **Figuren 6A und 6B** zeigen die Ergebnisse von Topfexperimenten mit Tomatenpflanzen vom Typ. Harzfeuer in Form von Säulendiagrammen. Dabei wurden Pflanzenwurzeln jeweils einzeln mit T. sp. HSA12 beziehungsweise mit dem Bacillus amyloliquefaciens Stamm FZB42 inokuliert und die Ergebnisse einerseits mit einer Kontrolle und andererseits mit Wurzeln verglichen, die mit einer Mischung von T. sp. HSA12 und FZB42 inokuliert wurden. Der Stamm FZB42 wurde als Produkt RhizoVital^{™} von der Firma AbiTEP Berlin erworben. Die **Figur 6A** zeigt einen Vergleich der Pflanzenhöhen in cm, während in der **Figur 6B** die Wurzelgewichte als jeweils messbare Größe für das Wurzelwachstum miteinander verglichen werden.

Die Pflanzenhöhe, gemessen in cm, steigt in der Reihenfolge Kontrolle < FZB42 < T. sp. HSA12 <<< Kombination aus T. sp. HSA12 und FZB42. Dabei ist der Unterschied zwischen der Pflanzenhöhe der mit einer Kombination aus T. sp. HSA12 und FZB42 behandelten Pflanze und der nächstgrößeren Pflanzenhöhe, nämlich der Pflanzenhöhe der nur mit T. sp. HSA12 behandelten Pflanze, deutlich größer als die jeweiligen Unterschiede zwischen den einzelnen Vergleichsproben.

Das Wurzelgewicht (Trockengewicht) in g steigt in der Reihenfolge Kontr. < T. sp. HSA12 < FZB42 <<< Kombination aus T. sp. HSA12 und FZB42. Dabei ist der Unterschied zwischen dem Wurzelgewicht der mit einer Kombination aus T. sp. HSA12 und FZB 42 behandelten Pflanze und dem nächsthöheren Wurzelgewicht, dem der nur mit FZB behandelten Pflanze, deutlich größer als die jeweiligen Unterschiede zwischen den einzelnen Vergleichsproben. Die **Figur 6C** zeigt fotografische Abbildungen der Wurzeln der unterschiedlich behandelten Pflanzen und der Kontrolle.

In beiden Fällen, sowohl bezüglich der Pflanzenhöhe als auch bezüglich des Wurzelgewichtes, liegt keine rein additive Wirkverstärkung durch die beiden in der Mischung enthaltenen Kulturen vor, vielmehr sind die entsprechenden Steigerungen der Pflanzenhöhe und des Wurzelgewichtes deutliche Zeichen für einen Synergismus.

Die **Tabelle 4a** zeigt die Sequenzen der Primerpaare für die Ermittlung des genetischen Fingerabdrucks des Pilzstammes der Gattung Trichoderma mit der Bezeichnung HSA12. Durch die Verfügbarkeit der kompletten Genomsequenz ist T. sp. HSA12 eindeutig charakterisiert und jeder Zeit zweifelsfrei identifizierbar, selbst wenn er anderen Produkten beigemischt ist. Ein Stamm ist durch seine Genomsequenz charakterisiert. Wird diese von zwei getrennten Kulturen bestimmt und ist identisch, handelt es sich per Definition auch um denselben Stamm. Dies träfe auch zu, wenn der Stamm ein zweites Mal aus der Natur isoliert würde. Bestehen aber auch nur geringe Abweichungen zwischen den Genomsequenzen, handelt es sich um einen anderen Stamm, ein weiteres Individuum der Spezies. Da Mikroorganismen und auch Mikropilze nur sehr wenige morphologische Charakteristika aufweisen, wird für die Zuordnung von Mikropilzen zu Spezies oder Gattungen ein Genabschnitt der ribosomalen DNA (rDNA) sequenziert und die erhaltene DNA-Sequenz mit in Datenbanken hinterlegten Referenzsequenzen verglichen, was als sogenanntes DNA-Barcoding, aus Schoch et al. Barcoding Consortium (2012). Nuclear ribosomal internal transcribed spacer as a universal DNA barcode marker for Fungi. PNAS 109 (16): 6241 - 6246**;** bekannt ist. Diese Analyse ergab mit einer Wahrscheinlichkeit von 98,8 % eine Zuordnung von HSA12 zur Spezies Trichoderma harzanium. Eine weitere und genauere Methode, der Vergleich der Gesamtgenomsequenzen (Phylogenomik) von HSA12 mit einem T. Harzanum Referenzstamm (Voucher Strain), ergab jedoch nur eine Übereinstimmung von 92 % aller Basenpaare. Bei Pilzen ist jedoch eine Übereinstimmung von mindestens 97 % erforderlich, um die DNA-Sequenzen eindeutig einer Spezies zuordnen zu können. Daher handelt es sich bei HSA12 definitiv nicht um Trichoderma harzianum, sondern womöglich um eine bisher unbekannte Trichoderma Spezies oder eine bekannte Trichoderma Spezies, deren Genomsequenz noch nicht bekannt ist. Daher wird der Pilzstamm als Pilzstamm HSA der Gattung Trichoderma oder Trichoderma spezies HSA12, kurz T. sp. HSA12, bezeichnet.

Da mittels DNA-Barcoding maximal Spezies, aber mit Sicherheit nur Gattungen bestimmt werden können und die Phylogenomik zur routinemäßigen Identifizierung von Individuen sehr aufwändig ist und auch erst wenige pilzliche Gesamtgenome sequenziert vorliegen, wird zur Erkennung von Individuen in der Routinediagnostik der "genetische Fingerabdruck" eingesetzt, siehe auch Geistlinger et al. "SSR Markers for Trichoderma virens: Their Evaluation and Application to Identify and Quantify Root-Endophytic Strains", Diversity 2015, 7, 360-384**.** Dazu werden sogenannte hypervariable Genomabschnitte herangezogen, sogenannte einfache Sequenzwiederholungen (SSRs = Simple sequence repeats), auch Mikrosatelliten oder SSR-Marker genannt. Folglich wurden aus dem T. sp. HSA12-Genom hypervariable Genomabschnitte analysiert und ein genetischer Fingerabdruck erstellt. Dieser genetische Fimgerabdruck dient einerseits zur Wiedererkennung des Individuums T. sp. HSA12, andererseits als Ausschlusskriterium zur Abgrenzung anderer Individuen aus der gleichen Spezies oder auch Gattung.

Die Erstellung des genetischen Fingerabdrucks erfolgt mittels Polymerase-Kettenreaktion (PCR). Dabei werden an jedem der fünfzehn HSA12-Loci die entsprechenden hypervariablen Genomabschnitte, auch SSR-Marker genannt, amplifiziert. Dabei legt ein Paar von sogenannten Primern, im Folgenden Primerpaar genannt, jeweils auf beiden DNA-Einzelsträngen den Startpunkt der DNA-Synthese fest, wodurch der zu vervielfältigende Bereich von beiden Seiten begrenzt wird. Mit Hilfe von DNA-Polymerase wird dann der festgelegte Abschnitt repliziert, wodurch die Amplifikation der DNA-Sequenzabschnitte erfolgt. Dabei ist die Reihe der verwendeten fünfzehn Primerpaare, im Folgenden Primer-Set genannt, spezifisch für die korrespondierenden Genomabschnitte des Pilzstamms Trichoderma sp. HSA12. In Tabelle 4a sind die dreißig Primersequenzen mit den entsprechenden SEQ.-Nr. 1 bis 30 aufgeführt, von denen jeweils zwei in der Sequenznummerierung (SEQ-Nr.) aufeinanderfolgende Sequenzen ein Primerpaar bilden.

An insgesamt fünfzehn HSA12-Loci (L1 bis L15) mit einfachen Sequenzwiederholungen (SSRs) entsprechend den Sequenzen mit den SEQ.-ID-Nr. 31 bis 45 wurde durch Polymerase-Kettenreaktion (PCR) die Fragmentlänge der jeweiligen Genomabschnitte bestimmt und mit anderen Trichoderma-Produkten verglichen. Diese Kombination an Fragmentlängen entsprechend **Tabelle 4b,** angegeben in Basenpaaren (bp), wurde in keinem anderen Isolat erhalten. Aus **Tabelle 4b** gehen auch die insgesamt fünfzehn sich wiederholenden Sequenzen mit den SEQ.-ID-Nr. 31 bis 45 und die jeweilige Anzahl der Sequenzwiederholungen (Repeat-Anzahl) in den Genomabschnitten hervor, die durch die insgesamt fünfzehn aus den Primersequenzen mit den SEQ.-ID-Nr. 1 bis 30 gebildeten Primerpaare amplifiziert werden.

**Tabelle 4a**

| Loci | Primerbezeichnungen | SEQ.-ID-Nr. | Primersequenzen 5'- 3' |
|---|---|---|---|
| L1 | HSA12S51GAA11f | 1 | |
| | HSA12S51GAA11r | 2 | |
| L2 | HSA12S52TCC12f | 3 | 5'-CAACAGCGAAGTGTTGATGG-3' |
| | HSA12S52TCC12r | 4 | |
| L3 | HSA12S53CAT11f | 5 | 5'-GTCTGGCTACATTGGCCTTC-3' |
| | HSA12S53CAT11r | 6 | |
| L4 | HSA12S54CTT15f | 7 | 5'-TCCTCCTCAATCACCTTGC-3' |
| | HSA 12S54CTT15r | 8 | |
| L5 | HSA 12S55AGT13f | 9 | |
| | HSA 12S55AGT13r | 10 | 5'-GCGTCATGTCCCCATCTATC-3' |
| L6 | HSA 12S56GAAGTGAAG7f | 11 | 5'-TTTCTTCGTGTTTCCCCATC-3' |
| | HSA 12S56GAAGTGAAG7r | 12 | |
| L7 | HSA 12S56GTTTGT8f | 13 | 5'-ATCAATAGACGGGGCATACG-3' |
| | HSA 12S56GTTTGT8r | 14 | |
| L8 | HSA 12S58CT14f | 15 | |
| | HSA 12S58CT14r | 16 | 5'-TATACCCCGCCTCAACAGTC-3' |
| L9 | HSA12S59TA12f | 17 | |
| | HSA12S59TA12r | 18 | |
| L10 | HSA 12S60TCAGG5f | 19 | |
| | HSA 12S60TCAGG5r | 20 | |
| L11 | HSA12S60CAG10f | 21 | |
| | HSA12S60CAG10r | 22 | |
| L12 | HSA12S61AGG6f | 23 | |
| | HSA12S61AGG6r | 24 | |
| L13 | HSA 12S63TGC7f | 25 | |
| | HSA 12S63TGC7r | 26 | 5'-ATGTACTTTTCCGCGTCCAG-3' |
| L14 | HSA12S66AGTGCC11f | 27 | |
| | HSA 12S66AGTGCC 11r | 28 | 5'-CCGGATTTATTTTGGTGGTG-3' |
| L15 | HSA 12S66AT17f | 29 | 5'-CATTTGGGGTGGGTATTCTG-3' |
| | HSA 12S66AT17r | 30 | |

**Tabelle 4b**

| SEQ-ID-Nr. | Tm [°C] | Locus | SEQ-ID-Nr. | Repeat-Anzahl | Fragmentlänge [bp] |
|---|---|---|---|---|---|
| 1 | 60,10 | L1 | 31 | (GAA)₁₁ | 488 |
| 2 | 60,30 | | | | |
| 3 | 60,19 | L2 | 32 | (TCC)₁₂ | 426 |
| 4 | 59,73 | | | | |
| 5 | 59,70 | L3 | 33 | (CAT)₁₁ | 365 |
| 6 | 60,29 | | | | |
| 7 | 60,20 | L4 | 34 | (CTT)₁₅ | 492 |
| 8 | 60,04 | | | | |
| 9 | 60,14 | L5 | 35 | (AGT)₁₃ | 204 |
| 10 | 60,31 | | | | |
| 11 | 59,91 | L6 | 36 | (GAAGTGAAG)₇ | 236 |
| 12 | 59,99 | | | | |
| 13 | 59,81 | L7 | 37 | (TTTGT)₈ | 287 |
| 14 | 59,99 | | | | |
| 15 | 59,99 | L8 | 38 | (GT)₁₄ | 289 |
| 16 | 59,96 | | | | |
| 17 | 60,28 | L9 | 39 | (TA)₁₂ | 468 |
| 18 | 60,01 | | | | |
| 19 | 60,09 | L10 | 40 | (TCAGG)₅ | 481 |
| 20 | 59,86 | | | | |
| 21 | 59,99 | L11 | 41 | (CAG)₁₀ | 460 |
| 22 | 60,20 | | | | |
| 23 | 59,34 | L12 | 42 | (AGG)₆ | 473 |
| 24 | 60,14 | | | | |
| 25 | 59,88 | L13 | 43 | (TGC)₇ | 401 |
| 26 | 60,13 | | | | |
| 27 | 60,02 | L14 | 44 | (AGTGCC)₁₁ | 306 |
| 28 | 60,05 | | | | |
| 29 | 60,04 | L15 | 45 | (AT)₁₇ | 33 |
| 30 | 59,93 | | | | |

## Patentansprüche

1. Pilzstamm der Gattung Trichoderma mit der Bezeichnung HSA12, welcher am 12.01.2018 unter der Patenthinterlegungsbezeichnung Nummer DSM 32722 beim Leibniz-Institut DSMZ- Deutsche Sammlung von Mikroorganismen und Zellkulturen GmbH hinterlegt wurde, oder Sporen davon.

2. Zusammensetzung, umfassend den Trichoderma-Spezies-Stamm HSA12 oder Sporen davon nach Anspruch 1.

3. Zusammensetzung nach Anspruch 2, ferner umfassend einen Träger.

4. Zusammensetzung nach Anspruch 2 oder 3, ferner umfassend eine Kombination des Trichoderma-Spezies-Stamms HSA12 mit anderen Mikroorganismen und/oder synthetischen oder biologischen Düngern und/oder Hilfsstoffen (Adjuvantien).

5. Verfahren zum Fördern und/oder zur Stabilisierung von Pflanzenwachstum und/oder zur Erhöhung der Erträge von Kulturpflanzen, umfassend die Inokulation des Bodens, der Wurzeln und/oder der oberirdischen Pflanzenteile mit dem Trichoderma-Spezies-Stamm HSA12 oder Sporen davon nach Anspruch 1 oder einer Zusammensetzung nach einem der Ansprüche 2 bis 4.

6. Verfahren nach Anspruch 5, **dadurch gekennzeichnet, dass** das Pflanzenwachstum unter abiotischem Stress stattfindet, ausgewählt aus Trockenstress, Kältestress und oxydativem Stress.

7. Verfahren nach Anspruch 6, **dadurch gekennzeichnet, dass** der abiotische Stress im Ergebnis eines, bezogen auf die jeweilige Region, nach vorne oder nach hinten verschobenen Aussaat- oder Auspflanzungstermins für die Kulturpflanze vorliegt.

8. Verfahren nach einem der Ansprüche 5 bis 7, **dadurch gekennzeichnet, dass** die Kulturpflanze eine landwirtschaftliche Pflanze ist.

9. Verfahren nach Anspruch 8, **dadurch gekennzeichnet, dass** die landwirtschaftliche Pflanze ausgewählt ist aus Tomatenpflanzen, Maispflanzen und Sojapflanzen.

10. Verfahren zur Verbesserung der Struktur und Gesundheit des Bodens oder zur Dekontamination oder Altlastensanierung eines toxische organische Substanzen enthaltenden Bodens oder Gewässers, umfassend eine Inokulation des Bodens oder des Gewässers mit dem Trichoderma-Spezies-Stamm HSA12 oder der Zusammensetzung nach einem der Ansprüche 2 bis 4 und Kultivieren des Trichoderma-Spezies-Stamms **HSA 12** und gegebenenfalls weiterer Stämme der Zusammensetzung im Boden oder im Gewässer.

11. Verfahren zur Stabilisierung oder Neuansiedlung gefährdeter oder erwünschter Wildpflanzenpopulationen, umfassend eine Inokulation des Bodens, der Wurzeln und/oder der oberirdischen Pflanzenteile der Wildpflanze mit dem Trichoderma-Spezies-Stamm HSA12 oder der Zusammensetzung nach einem der Ansprüche 2 bis 4.

12. Primerpaar-Satz für die Amplifikation von Mikrosatelliten-Loci des Genoms des Pilzstamms nach Anspruch 1 zur Bestimmung von molekularen Markern und zur Identifizierung des Pilzstamms, wobei die jeweiligen Primerpaare aus den Primern mit den Primersequenzen SEQ-ID-Nr. 1 und 2; 3 und 4; 5 und 6; 7 und 8; 9 und 10; 11 und 12; 13 und 14; 15 und 16; 17 und 18; 19 und 20; 21 und 22; 23 und 24; 25 und 26; 27 und 28; 29 und 30 gebildet werden.

13. Verfahren zur Bestimmung des Pilzstamms nach Anspruch 1, umfassend die Verfahrensschritte
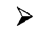 Gewinnung der DNA aus einer Probe, die den Pilz enthält,
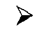 Amplifikation von Genomabschnitten als Mikrosatelliten-Loci zur Bestimmung von molekularen Markern mittels der Primerpaare des Primerpaar-Satzes nach Anspruch 12 und der Polymerase Kettenreaktion (PCR),
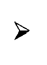 Bestimmung der Fragmentlänge der jeweiligen Genomabschnitte, angegeben in Basenpaaren (bp), und/oder der Art sich wiederholenden Sequenzen und/oder der jeweiligen Anzahl der Sequenzwiederholungen (Repeat-Anzahl) in den Genomabschnitten und Vergleich mit den entsprechenden Eigenschaften des Pilzstamms nach Anspruch 1.

14. Marker-Satz zur Bestimmung des Pilzstamms nach Anspruch 1, amplifiziert durch ein Primerpaar-Satz nach Anspruch 12, umfassend die Sequenzen mit den SEQ-ID-Nr. 31 bis 45.

## Claims

1. Fungal strain of the genus Trichoderma with the designation HSA12 that was deposited at the Leibniz Institute DSMZ-German Collection of Microorganisms and Cell Cultures GmbH under the patent deposit designation number DSM 32722 on 12 January 2018, or spores thereof.

2. Composition comprising the Trichoderma species strain HSA12 or spores thereof according to Claim 1.

3. Composition according to Claim 2, further comprising a carrier.

4. Composition according to Claim 2 or 3, further comprising a combination of the Trichoderma species strain HSA12 with other microorganisms and/or synthetic or biological fertilizers and/or adjuvants.

5. Method for promoting and/or for stabilizing plant growth and/or for increasing the yields of cultivated plants, comprising the inoculation of the soil, the roots and/or the epigeal plant parts with the Trichoderma species strain HSA12 or spores thereof according to Claim 1 or a composition according to any of Claims 2 to 4.

6. Method according to Claim 5, **characterized in that** the plant growth is taking place under abiotic stress selected from dry stress, cold stress and oxidative stress.

7. Method according to Claim 6, **characterized in that** the abiotic stress is present for the cultivated plant as a result of a sowing or planting date moved forward or backward based on the particular region.

8. Method according to any of Claims 5 to 7, **characterized in that** the cultivated plant is an agricultural plant.

9. Method according to Claim 8, **characterized in that** the agricultural plant is selected from tomato plants, maize plants and soya plants.

10. Method for improving the structure and health of the soil or for decontaminating or cleaning up a soil or body of water that contains toxic organic substances, comprising inoculation of the soil or the body of water with the Trichoderma species strain HSA12 or the composition according to any of Claims 2 to 4 and cultivation of the Trichoderma species strain HSA12 and, if applicable, further strains of the composition in the soil or in the body of water.

11. Method for stabilizing or resettling endangered or desirable wild-plant populations, comprising inoculation of the soil, the roots and/or the epigeal plant parts of the wild plant with the Trichoderma species strain HSA12 or the composition according to any of Claims 2 to 4.

12. Primer-pair set for the amplification of microsatellite loci of the genome of the fungal strain according to Claim 1 for determining molecular markers and for identifying the fungal strain, wherein the respective primer pairs are formed from the primers having the primer sequences SEQ ID Nos. 1 and 2; 3 and 4; 5 and 6; 7 and 8; 9 and 10; 11 and 12; 13 and 14; 15 and 16; 17 and 18; 19 and 20; 21 and 22; 23 and 24; 25 and 26; 27 and 28; 29 and 30.

13. Method for determining the fungal strain according to Claim 1, comprising the method steps of
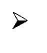 obtaining the DNA from a sample containing the fungus,
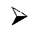 amplifying genomic segments in the form of microsatellite loci for determination of molecular markers by means of the primer pairs of the primer-pair set according to Claim 12 and the polymerase chain reaction (PCR),
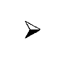 determining the fragment length of the respective genomic segments, reported in base pairs (bp), and/or the nature of the repeating sequences and/or the respective number of sequence repeats (repeat number) in the genomic segments and making a comparison with the corresponding properties of the fungal strain according to Claim 1.

14. Marker set for determining the fungal strain according to Claim 1, amplified by a primer-pair set according to Claim 12, comprising the sequences having SEQ ID Nos. 31 to 45.

## Revendications

1. Souche fongique du genre Trichoderma ayant la désignation HSA12, qui a été déposée sous le numéro de dépôt de brevet DSM 32722 le 12 janvier 2018 auprès de l'Institut Leibniz DSMZ- Deutsche Sammlung von Mikroorganismen und Zellkulturen GmbH (Collection allemande de micro-organismes et de culture cellulaire), ou spores de cette souche.

2. Composition comprenant la souche de l'espèce Trichoderma HSA12 ou des spores de cette souche selon la revendication 1.

3. Composition selon la revendication 2, comprenant en outre un support.

4. Composition selon la revendication 2 ou 3, comprenant en outre une combinaison de la souche de l'espèce Trichoderma HSA12 avec d'autres micro-organismes et/ou des engrais synthétiques ou biologiques et/ou des substances auxiliaires (adjuvants).

5. Procédé de promotion et/ou de stabilisation de la croissance des plantes et/ou d'augmentation de la production de plantes de culture, comprenant l'inoculation, dans le sol, les racines et/ou les parties de plante aériennes, de la souche de l'espèce Trichoderma HSA12 ou de spores de cette souche selon la revendication 1, ou d'une composition selon l'une des revendications 2 à 4.

6. Procédé selon la revendication 5, **caractérisé en ce que** la croissance des plantes a lieu sous un stress abiotique, choisi parmi le stress de sécheresse, le stress de froid et le stress oxydatif.

7. Procédé selon la revendication 6, **caractérisé en ce que** le stress abiotique est une conséquence d'une date de semis ou de repiquage décalé vers l'avant ou vers l'arrière, en fonction de la région considérée.

8. Procédé selon l'une des revendications 5 à 7, **caractérisé en ce que** la plante de culture est une plante agricole.

9. Procédé selon la revendication 8, **caractérisé en ce que** la plante agricole est choisie parmi les plantes de tomate, les plantes de maïs et les plantes de soja.

10. Procédé d'amélioration de la structure et de la santé du sol, ou de décontamination ou de remédiation de la contamination d'un sol ou d'une masse d'eau contenant des substances organiques toxiques, comprenant l'inoculation, dans le sol ou dans la masse d'eau, de la souche de l'espèce Trichoderma HSA12 ou de la composition selon l'une des revendications 2 à 4, et la culture de la souche de l'espèce Trichoderma HSA12 et éventuellement d'autres souches de la composition dans le sol ou dans la masse d'eau.

11. Procédé de stabilisation ou de recolonisation de populations de plantes sauvages en danger ou souhaitées, comprenant une inoculation, dans le sol, les racines et/ou les parties de plante aériennes de la plante sauvage, de la souche de l'espèce Trichoderma HSA12 ou de la composition selon l'une des revendications 2 à 4.

12. Jeu de paires d'amorces pour l'amplification de loci de microsatellites du génome de la souche fongique selon la revendication 1 pour déterminer des marqueurs moléculaires et pour identifier la souche fongique, les paires d'amorces respectives étant formées à partir des amorces présentant les séquences d'amorces SEQ ID NO : 1 et 2 ; 3 et 4 ; 5 et 6 ; 7 et 8 ; 9 et 10 ; 11 et 12 ; 13 et 14 ; 15 et 16 ; 17 et 18 ; 19 et 20 ; 21 et 22 ; 23 et 24 ; 25 et 26 ; 27 et 28 ; 29 et 30.

13. Procédé de détermination de la souche fongique selon la revendication 1, comprenant les étapes suivantes :
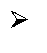 obtention de l'ADN à partir d'un échantillon contenant le champignon,
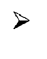 amplification de segments génomiques sous forme de loci de microsatellites pour déterminer des marqueurs moléculaires à l'aide des paires d'amorces du jeu de paires d'amorces selon la revendication 12 et par une réaction en chaîne par polymérase (PCR),
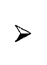 détermination de la longueur des fragments des segments génomiques respectifs, indiquée en paires de bases (pb) et/ou à la manière de séquences répétitives et/ou du nombre respectif de répétitions de séquence (nombre de repeats) dans les segments génomiques et comparaison avec les propriétés correspondantes de la souche fongique selon la revendication 1.

14. Jeu de marqueurs pour déterminer la souche fongique selon la revendication 1, amplifiée par un jeu de paires d'amorces selon la revendication 12, comprenant les séquences SEQ ID NO : 31 à 45.
